Europäisches Patentamt

European Patent Office

Office européen des brevets

(11) Veröffentlichungsnummer: **0 674 906 A2**

# (12) EUROPÄISCHE PATENTANMELDUNG

(21) Anmeldenummer: **95104556.6**

(22) Anmeldetag: **28.03.95**

(51) Int. Cl.⁶: **A61K 38/49**, //(A61K38/49, 38:48)

(30) Priorität: **30.03.94 DE 4411143**

(43) Veröffentlichungstag der Anmeldung: **04.10.95 Patentblatt 95/40**

(84) Benannte Vertragsstaaten: **AT BE CH DE DK ES FR GB IT LI NL SE**

(71) Anmelder: **IMMUNO AG**
**Industriestrasse 67**
**A-1221 Wien (AT)**

(72) Erfinder: **Eibl, Johann, Dr.**
**Gustav Tschermak Gasse 2**
**A-1180 Wien (AT)**
Erfinder: **Philapitsch, Anton**
**Hans Kudlich Gasse 5**
**A-2490 Ebenfurt (AT)**
Erfinder: **Schwarz, Hans Peter, Doz.**
**Schindlergasse 32**
**A-1180 Wien (AT)**

(74) Vertreter: **Kolb, Helga, Dr. Dipl.-Chem. et al**
**Hoffmann, Eitle & Partner,**
**Patent-und Rechtsanwälte,**
**Arabellastrasse 4**
**D-81925 München (DE)**

(54) Thrombosemittel, das Plasmin und einen Plasminogenaktivator enthält.

㊗ Es wird eine pharmazeutische Zusammensetzung mit thrombolytischer Wirkung beschrieben, die Plasmin und einen Plasminogenaktivator enthält.

Mit dieser Zusammensetzung ist eine einfach und sicher durchführbare Therapie thrombotischer Erscheinungsformen möglich. Sie eignet sich sowohl zur systemischen als auch zur lokalen Applikation am Ort der Thrombose.

EP 0 674 906 A2

Die Erfindung betrifft eine pharmazeutische Zusammensetzung mit thrombolytischer Wirkung (Thrombosemittel).

Unter Thrombose versteht man eine krankhafte Blutgerinnung innerhalb von Gefäßen, meist von Venen, aber auch von Arterien, die zum Gefäßverschluß führt. Als Folge treten bei der Venen-Thrombose Zeichen der Blutstauung auf, wie bläuliche Verfärbung und Ödem, sowie Schmerzen und Mißempfindungen. Eine Thrombose ist durch die Gefahr des Weitertransportes von Gerinnseln in Organe, wie Niere, Hirn oder Lunge (Embolie) besonders gefährlich.

Ursache von Thrombosen sind Schäden der Gefäßwand, die zur Anlagerung von Thrombocyten führen, sowie Störungen in der Regulation der Blutgerinnung (z.B. durch Mangel an Antithrombin III) sowie Veränderungen der Fließeigenschaften des Blutes mit Wirbelbildung und Zirkulationsbehinderung, wie z.B. bei längerer Bettlägerigkeit.

Die Behandlung der Thrombose kann auf 2 verschiedenen Wegen erfolgen, nämlich (1) durch eine medikamentöse Auflösung des Gerinnsels (durch Thrombolyse), und (2) insbesondere bei nicht auflösbaren Gerinnseln durch einen operativen Eingriff. Daneben besteht selbstverständlich auch die Möglichkeit eine Kombination von (1) und (2).

Insbesondere zur Behandlung von Patienten, die zu Thrombosen neigen, bietet sich die vorstehend als (1) bezeichnete medikamentöse Auflösung, d.h. die Thrombolysetherapie an. Dabei wird intravaskuläres Fibrin aufgelöst und ein Thrombus verkleinert bzw. eliminiert. Der Erfolg einer Thrombolysetherapie hängt dabei nicht zuletzt von einer raschen Auflösung eines Thrombus ab, damit die Perfusion eines Blutgefäßes zu einem möglichst frühen Zeitpunkt wieder hergestellt werden kann.

Venöse und arterielle Thromben, die embolisch oder am Ort des thrombotischen Blutgefäßverschlusses entstehen, enthalten Fibrin. Dieses Fibrin kann durch das fibrinolytisch wirksame Enzym Plasmin lysiert werden, wodurch der Thrombus aufgelöst wird.

Plasmin ist die Bezeichnung für das proteolytische Enzym, eine Serin-Proteinase, das aus Fibrin bestehende Blutgerinnsel zu löslichen Produkten (Fibrinopeptiden) abbauen kann, also als Fibrinolyticum (Thrombolyticum) wirksam ist. Bei Bedarf entsteht Plasmin am Ort des Gerinnsels aus der inaktiven Vorstufe Plasminogen, d.h. dem Zymogen des Plasmins, das in einer Konzentration von etwa 200 mg/ml Blut-Plasma vorkommt.

Die Aktivierung des Plasminogens zur Plasmin erfolgt durch verschiedene Plasma-Faktoren oder durch zu den Serin-Proteinasen gehörenden speziellen Plasminogen-Aktivatoren, wie Urokinase oder den Gewebs-PA (tPA, tissue plasminogen activator), der auch mit dem Gefäßwand-PA identisch ist. tPA besitzt ein Molekulargewicht von 72000 und setzt sich aus verschiedenen Strukturbausteinen (Domänen) zusammen, die durch Disulfidbrücken stabilisiert werden, nämlich eine Finger-, Wachstumsfaktor-, zwei Kringel- und eine Serin-Proteinase-Domäne. Mit Hilfe der Kringel-Domänen bindet der tPA an Fibrin-Gerinnsel und wird dadurch dazu aktiviert, das gleichfalls dort gebundene Plasminogen zu spalten.

So wird z.B. Streptokinase, ein exogener Aktivator aus Streptokokken, der selbst weder Kinase-typische oder proteolytische Aktivität besitzt, erst nach Komplexbildung mit Plasminogen enzymatisch auf Plasminogen ein (J. Biol. Chem 253 (1987) 1090-1094).

Im Rahmen einer Thrombolysetherapie ist es deshalb üblich, ein als Plasminogenaktivator wirksames Enzym zu verabreichen; ein solches sogenanntes thrombolytisch wirksames Enzym kann beispielsweise tPA, Urokinase, pro-Urokinase, Streptokinase und Staphylokinase sein.

V.V. Kakkar und F.M. Scully, Haemostasis 18: Suppl. 1 (1988) 127 - 138 beschreiben die Thrombolysebehandlung von Patienten durch eine Infusion von Plasminogen und einer anschließenden Infusion von Streptokinase zur Aktivierung des Plasminogens in vivo; zum Vergleich wurden Patienten mit Streptokinase allein behandelt. Dabei hat sich gezeigt, daß die konsekutive Verabreichung von Plasminogen und Plasminogenaktivator der alleinigen Verabreichung von Streptokinase überlegen war.

Von A. Schoppmann et al., Haemostasis 18: Suppl. 1 (1988) 157-163 wird zur Thrombolysetherapie die Verabreichung von Lys-Plasminogen anstelle des nativen Glu-Plasminogens vorgeschlagen. Lys-Plasminogen ist ein in der Literatur verwendeter Sammelbegriff für proteolytisch modifizierte Formen des nativen Plasminogens (Glu-Plasminogens), die aus diesem durch Abspaltungen eines Polypeptids von NH2-Terminus erhalten werden. Als N-therminale Aminosäure der heute bekannten Spezies des Lys-Plasminogens wurden bisher Lysin, Methionin und Valin nachgewiesen. Im Gegensatz zum Molekulargewicht von Glu-Plasminogen, das bei 90000 - 94000 liegt, werden für Lys-Plasminogen Werte von etwa 80000 angegeben. Lys-Plasminogen hat den Vorteil, das es mit einer höheren Affinität an Fibrin adsorbiert und mit einem Plasminogenaktivator rascher zu Plasmin umgewandelt werden kann. Lys-Plasminogen wird beispielsweise aus Plasma hergestellt und zur Virusinaktivierung behandelt, um die Übertragung von infektiösen Agentien auszuschließen.

Die kombinierte Verabreichung von Plasminogen mit einem Plasminogenaktivator kann aber auch in Form eines Komplexes aus den beiden Komponenten erfolgen. Y. Takada und A. Takada, Thrombosis Research 54 (1989) 133 bis 139 beschreiben die Bildung eines equimolaren Komplexes aus Streptokinase und Plasminogen. Dieser Komplex führt in Gegenwart von Fibrin zu einer raschen Bildung von Plasmin. In Abwesenheit von Fibrin oder Fibrinogen kommt es jedoch nur zu einer langsamen und unzureichenden Umwandlung des Streptokinase-Plasminogen-Komplexes zu einem Streptokinase-Plasmin-Komplex.

Aus der EP-B-0337817 ist ebenfalls ein Komplex aus Plasminogen und einem Plasminogenaktivator, nämlich Staphylokinase, bekannt. Der Komplex wird im equimolaren Verhältnis hergestellt und isoliert, wodurch keine freie Staphylokinase mehr anwesend ist. Bei der Komplexbildung kommt es zur teilweisen Umwandlung von Plasminogen zu Plasmin, weshalb effektiv ein Staphylokinase/Plasminogen (Plasmin)-Komplex vorliegt.

Wenn eine direkte Verabreichung des fibrinolytisch wirksamen Enzyms Plasmin gewünscht wird, so wird das Plasminogen in vitro zu Plasmin umgewandelt. Dabei wird aus Gründen des autolytischen Abbaus das Plasmin zumeist durch Zusatz üblicher Stabilisatoren stabilisiert. Die Herstellung von Plasmin kann beispielsweise wie in WO-A-93/07893 beschrieben erfolgen. Plasminogen wird mit einem immobilisierten Plasminogenaktivator in Kontakt gebracht, um auf die Weise das aktive Plasmin zu erhalten; dabei wird darauf geachtet, daß die Plasminpräparation keinen Gehalt an Plasminogenaktivator mehr aufweist.

Aufgabenstellung der vorliegenden Erfindung ist die Bereitstellung eines Plasmin enthaltenden pharmazeutischen Präparates mit verbesserter thrombolytischer Wirkung, das einfach und kostengünstig herzustellen ist, und auf einfache und sichere Weise appliziert werden kann.

Diese Aufgabenstellung wird mit der vorliegenden Erfindung gelöst.

Gegenstand der Erfindung ist ein pharmazeutisches Präparat mit thrombolytischer Wirkung nach Anspruch 1, das Plasmin und einen Plasminogenaktivor, der zumindest teilweise, vorzugsweise zu mindestens 30 %, und in erster Linie zu mindestens 50 % freier Form, also in nicht-komplexgebundener Form, vorliegt, zusammen mit üblichen pharmazeutischen Hilfs- und/oder Trägerstoffen, enthält.

Bevorzugte Ausführungsformen davon sind Gegenstand der Ansprüche 2 bis 12.

Weiterer Gegenstand ist die Verwendung nach Anspruch 13 einer erfindungsgemäßen Zusammensetzung zur systemischen oder lokalen Applikation, und die Verwendung nach Anspruch 14 von Plasmin zusammen mit Plasminogenaktivator und gegebenenfalls Plasminogen(en) zur Herstellung eines Arzneimittels.

Bevorzugte Ausführungsformen dieser Verwendungen sind Gegenstand der Ansprüche 15 bis 17.

Weiterer Gegenstand der Erfindung ist ein Verfahren gemäß Anspruch 18 zur Herstellung einer erfindungsgemäßen Zusammensetzung, das dadurch gekennzeichnet ist, daß man die Komponenten Plasminogen und/oder Plasmin mit einem Plasminogenaktivator vermischt und gegebenenfalls inkubiert, um Plasmin zu bilden.

Zweckmäßige Ausgestaltungen dieses Verfahrens sind Gegenstand der Ansprüche 19 bis 25.

Eine zweckmäßige Ausgestaltung der erfindungsgemäßen Zusammensetzungen, Verwendungen und/oder Verfahren ist Gegenstand von Patentanspruch 26.

Ein weiterer Gegenstand der vorliegenden Erfindung ist ein Verfahren gemäß Patentanspruch 27 zur Herstellung eines Plasmins für eine erfindungsgemäße Zusammensetzung, erfindungsgemäße Verwendung oder ein erfindungsgemäßes Verfahren.

Zweckmäßige Ausgestaltungen davon sind Gegenstand der Ansprüche 28 bis 30.

Weiterer Gegenstand ist auch ein Verfahren gemäß Patentanspruch 31 zur Behandlung von thrombotischen Erscheinungen bei Säugern.

Unter Plasmin wird dabei erfindungsgemäß ein "aktives Plasmin" verstanden, d.h. ein fibrinolytisch wirkendes Plasmin, z.B. ein aus seinem zymogenen Plasminogen in vitro erhaltenes und gegebenenfalls stabilisiertes Plasmin.

Überraschenderweise wurde gefunden, daß die erfindungsgemäße Zusammensetzung, die neben Plasmin einen Plasminogenaktivator in zumindest teilweise freier Form enthält, eine hervorragende thrombolytische Wirkung zeigt, die der unter alleiniger Verwendung von Plasmin zu erzielenden Wirkung überlegen ist.

Dies war z.B. aufgrund des vorstehend genannten Standes der Technik, wonach z.B. Streptokinase, ein exogener Aktivator, erst nach Komplexbildung mit Plasmin(ogen) aktivierend auf Plasminogen einwirkt, nicht zu erwarten.

Mit der erfindungsgemäßen Zusammensetzung ist es deshalb möglich, nicht nur den unmittelbaren fibrinolytischen Effekt von (aktivem) Plasmin auszunutzen, sondern auch den nachhaltigen unterstützenden Effekt eines Plasminogenaktivators, der aufgrund seiner zumindest teilweise ungebundenen, also nicht komplexgebundenen Form leicht das im Körper bzw. im Thrombus vorhandene endogene Plasminogen aktivieren kann. Es muß als überraschend angesehen werden, daß die erfindungsgemäße Zusammensetzung, die Plasmin und einen Plasminogenaktivator, der zumindest teilweise in freier Form vorliegt, enthält, im Vergleich zu einer alleinigen Anwendung von Plasmin zu wesentlich effektiveren Ergebnissen und vor allem zu einer rascheren Thrombolyse führt.

An Patienten mit zerebralen Blutgerinnseln wurde auch festgestellt, daß die erfindungsgemäße Zusammensetzung die Erfolgsquote einer Thrombolysetherapie signifikant verbessert.

Die erfindungsgemäße Zusammensetzung kann systemisch oder lokal, z.B. direkt am Ort der Thrombose bzw. Embolie, appliziert werden.

Erfindungsgemäß bevorzugt ist es, das Präparat direkt am Ort der Thrombose bzw. Embolie zu applizieren, z.B. mit Hilfe eines Katheters, der in das entsprechende Blutgefäß, z.B. intraarteriell, eingeführt wird. Auf diese Weise lassen sich Nebenwirkungen, die mit einer systemischen Verabreichung von Plasmin verbunden sein können, vermeiden.

Gegenstand der Erfindung ist deshalb auch die Verwendung einer erfindungsgemäßen, Plasmin und Plasminogenaktivator enthaltenden Zusammensetzung zur systemischen oder vorzugsweise lokalen Applikation (am Ort der Thrombose bzw. Embolie) bei der Behandlung und Prophylaxe von Thrombose bzw. Embolie und verwandten Erscheinungsformen.

Weiterer Gegenstand der Erfindung ist die Verwendung von Plasmin zusammen mit Plasminogenaktivator zur Herstellung eines Applikationssystems, z.B. eines Arzneimittels, zur systemischen oder vorzugsweise lokalen Applikation bei der Behandlung und Prophylaxe von Thrombose bzw. Embolie und verwandten Erscheinungsformen.

Weiterer Gegenstand der Erfindung ist die Verwendung einer erfindungsgemäßen Zusammensetzung oder von Plasmin und Plasminogenaktivator zur Herstellung oder Bereitstellung eines Applikationssystems, z.B. auf der Basis einer Vorrichtung zur lokalen Applikation bei der Behandlung und Prophylaxe von Thrombose bzw. Embolie und ähnlichen Erscheinungsformen.

Gegenstand der Erfindung ist ebenfalls auch ein Verfahren zur Behandlung und Prophylaxe von thrombotischen Erscheinungen bei Säugern (Mensch und Tier), nach dem man eine erfindungsgemäße Zusammensetzung, oder die Komponenten Plasmin, Plasminogenaktivator und gegebenenfalls Plasminogen(e) systemisch oder lokal, z.B. am Ort der Thrombose bzw. Embolie, appliziert. Vorzugsweise wird die Behandlung vor, während oder nach einer Thrombolysetherapie vorgenommen.

Der Plasminogenaktivator wird erfindungsgemäß zumindest teilweise in freier Form, also nicht zur Gänze als Komplexe mit Plasmin und/oder Plasminogen, eingesetzt.

Vorzugsweise ist die Vorrichtung zur lokalen Applikation eine Kanüle oder ein Katheter zur Einführung in ein Blutgefäß. Eine solche Kanüle oder Katheter kann ein(e) üblicherweise zur Einführung in ein Blutgefäß verwendete Kanüle oder Katheter mit einer dafür geeigneten und üblichen Form und aus einem dafür geeigneten und üblichen Material sein.

Die Vorrichtung, die vorzugsweise eine Kanüle oder ein Katheter zur Einführung in einem Blutgefäß ist, kann die erfindungsgemäße Zusammensetzung oder die Komponenten Plasmin, Plasminogenaktivator, oder die entsprechenden Ausgangsprodukte zu deren Herstellung enthalten. Alternativ und vorzugsweise wird die erfindungsgemäße Zusammensetzung oder Applikationssystem, z.B. die Komponenten Plasminogen und/oder Plasmin sowie Plasminogenaktivator, gleichzeitig oder gegebenenfalls nacheinander, durch die Vorrichtung, insbesondere Kanüle oder Katheter, hindurchgeführt werden, wobei die Vorrichtung vorzugsweise so in den Körper eingeführt ist, daß die erfindungsgemäße Zusammensetzung oder das Applikationssystem den Ort der Thrombose oder deren unmittelbare Umgebung erreicht.

Vorzugsweise enthält die erfindungsgemäße Zusammensetzung zusätzlich noch ein oder mehrere Plasminogene, und insbesondere ein Lys-Plasminogen.

Gleiches gilt bei einer Verwendung der Einzelkomponenten Plasmin und Plasminogenaktivator; auch hier wird vorzugsweise zusätzlich noch ein oder mehrere Plasminogene mitverwendet, wobei die Applikation vorzugsweise gleichzeitig, oder aber auch in beliebiger, kurzzeitig aufeinanderfolgender Reihenfolge erfolgen kann.

Als Träger- und/oder Hilfsstoffe für die pharmazeutische Zusammensetzung oder als Träger (flüssigkeit) für die Applikation der Einzelkomponenten, kommen für derartige Zusammensetzungen und Anwendungen übliche und allgemein bekannte pharmazeutische Trägersubstanzen, Verdünner und/oder Hilfsmittel in Frage.

Die nachfolgend angegebenen bevorzugten Mengen und Mengenverhältnisse von Plasmin, Plasminogenaktivator und/oder Plasminogenen beziehen sich auf die erfindungsgemäße pharmazeutische Zusammensetzung, aber auch auf die Applikation der Einzelkomponenten, z.B. bei der erfindungsgemäßen Verwendung zusammen mit einer an den Ort der Thrombose bzw. Embolie einzuführenden Vorrichtung, vorzugsweise einer Kanüle oder eines Katheters.

Das molare Verhältnis Plasmin/Plasminogenaktivator beträgt vorzugsweise 1/0.01 bis 1/3.

4

Plasmin und tPA werden vorzugsweise im Verhältnis 1:0.1 bis 1:1000, und insbesondere 1:0.5 bis 1:125 und in erster Linie von ca. 1:4 (CU/µg) eingesetzt.

Die Einheit CU entspricht dabei der caseinolytischen Aktivität von Plasmin, welches von Casein Fragmente abspaltet, die in Trichloressigsäure löslich sind und durch spektrophotometrische Bestimmung der Extinktion bei 280 nm erfaßbar sind.

Der Nachweis für einen Plasminogenaktivator in freier bzw. nicht komplexierter Form in Plasminpräparat kann dabei durch übliche Methoden, wie z.B. durch Gel-Chromatographie, SDS-Elektrophorese (unter nicht-reduzierenden Bedingungen) oder durch Adsorption des Plasmins an immobilisiertem Lysin erfolgen.

Wenn erfindungsgemäß zusätzlich zum Plasmin und Plasminogenaktivator auch noch ein oder mehrere Plasminogene verwendet werden, so beträgt das molare Verhältnis von Plasmin zu Plasminogenen vorzugsweise 1:0.01 bis 1:1.

Die erfindungsgemäße Zusammensetzung enthält das Plasmin vorzugsweise in einer Menge von 1 bis 95 Gewichtsteilen, bezogen auf das Gewicht der gesamten Zusammensetzung, und insbesondere in einer Menge von 10 bis 30 Gewichtsteilen.

Die Mengen an Plasminogenaktivator und gegebenenfalls zusätzlichen Plasminogenen ergeben sich daraus aus den vorstehend genannten bevorzugten Mengenverhältnissen.

Die zu verabreichende Dosis der erfindungsgemäßen Zusammensetzung richtet sich sowohl bei der systemischen als auch bei der lokal erfolgenden Anwendung nach der Schwere der Erkrankung, dem Allgemeinbefinden des Patienten und insbesondere auch nach der Thrombose-Anamnese, d.h. z.B. nach der Häufigkeit und den Eigenschaften bisher auftretender thrombotischer Erscheinungen.

Gleiches gilt auch für eine Applikation der Einzelkomponenten, z.B. bei einer erfindungsgemäßen Verwendung zusammen mit einem Katheter oder einer Kanüle.

Als Plasminogenaktivator kann ein Aktivator, eine Kombination und/oder Chimäre eingesetzt werden, vorzugsweise ausgewählt aus der Gruppe tPA, Urokinase, Pro-Urokinase und Streptokinase. Die Plasminaktivatoren liegen in der erfindungsgemäßen Zusammensetzung zumindest teilweise in nicht-komplexierter Form vor.

Die erfindungsgemäße Zusammensetzung kann nach einem an sich bekannten, für derartige Zusammensetzungen üblicherweise verwendeten Verfahren durch Vermischen der Komponenten Plasmin, Plasminogenaktivator und gegebenenfalls ein oder mehrere Plasminogene in einem dafür geeigneten Gefäß, vorzugsweise unter Rühren, erfolgen. Je nach der beabsichtigten Dareichungsform kann die Vermischung in dem bereits als Träger, z.B. für eine Infusionslösung, geeigneten Verdünnungs(lösungsmittel) erfolgen, oder aber auch in einem pharmazeutisch annehmbaren Lösungsmittel, gegebenenfalls zusammen mit anderen Hilfs- und/oder Trägerstoffen zur nachträglichen Haltbarmachung erfolgen. Das auf diese Weise erhaltene Präparat oder die weiter zu verarbeitete Mischung wird vorzugsweise durch Zugabe eines geeigneten, an sich bekannten Stabilisators, wie z.B. Phospholipide, Kohlenhydrate, Albumin, stabilisiert; je nach der gewünschten Dareichungsform können sich an die Herstellung durch Vermischung und nachfolgende Stabilisierung dann noch weitere Verfahrensschritte anschließen, wie z.B. vorzugsweise eine Lyophilisierung.

Überraschenderweise wurde gefunden, daß die Inkubation von Plasmin und/oder Plasminogen mit einem Plasminogenaktivator zur Herstellung der erfindungsgemäßen Zusammensetzung in einfacher Weise vorgenommen werden kann; diese Inkubation ist dabei zumeist von sehr kurzer Dauer, so daß eine extensive Komplexbildung vermieden wird. Dies ist insbesondere deshalb überraschend, weil, wie dies einleitend bei der Besprechung des Standes der Technik angezeigt wurde, bestimmte exogene Aktivatoren, wie z.B. Streptokinase, erst nach Komplexbildung enzymatisch auf Plasminogen einwirken.

Vorteilhafterweise wird deshalb die erfindungsgemäße Zusammensetzung erst kurz vor der beabsichtigten Applikation im Rahmen einer Thrombolysetherapie oder auch während der Thrombolysetherapie hergestellt, insbesondere dann, wenn es z.B. in Form einer Infusionslösung oder zusammen mit einer an den Ort der Thrombose einzuführenden Vorrichtung, z.B. einer Kanüle oder einem Katheter, appliziert wird. Aufgrund der kurzen Reaktionszeiten kann das erfindungsgemäße Präparat dabei sogar während der Thrombolysetherapie hergestellt werden, z.B. innerhalb einer an den Ort der Thrombose eingeführten Vorrichtung, wie z.B. einer Kanüle oder eines Katheters, dem erfindungsgemäß die einzelnen Komponenten gleichzeitig oder rasch hintereinander, insbesondere in einem dafür geeigneten Verdünnungsmittel, zugeführt werden. Die Zufuhr in die Vorrichtung (Katheter oder Kanüle) soll dabei vorzugsweise mit einer solchen Geschwindigkeit erfolgen, daß eine Reaktionszeit von mindestens 30 sec., und insbesondere von mindestens 1 Minute also eine ausreichende Inkubationszeit erreicht wird, bis die Zusammensetzung, d.h. das dann bereits erfindungsgemäße fertige Präparat, den Ort der Applikation erreicht.

Wie bereits vorstehend angegeben, wird, wenn die erfindungsgemäße Zusammensetzung durch Reaktion eines Plasminogens und Plasminogenaktivators hergestellt wird, insbesondere Lys-Plasminogen verwendet. Das Plasminogen ist vorzugsweise Virus-inaktiviert. Das Plasminogen wird mit einem Plasminogenaktivator in Kontakt gebracht und vorzugsweise bei einer Temperatur von 15 bis 37 °C, und insbesondere bei Raumtemperatur, in der Regel mindestens 30 sec., und vorzugsweise mindestens 1 Minute, bis zum Erreichen einer gewünschten Plasminaktivität inkubiert.

Die Inkubationszeit richtet sich dabei nicht nur nach der Menge des Plasminogens und des Plasminogenaktivators, sondern z.B. auch nach dem Ausmaß der Komplexbildung mit dem Plasminogenaktivator, die aber erfindungsgemäß vorzugsweise weitgehend vermieden werden soll.

Optimierungsversuche haben gezeigt, daß bereits nach 2 Minuten Inkubation bei 37 °C mindestens 50 % des eingesetzten Lys-Plasminogens (in einer Konzentration von 25 CU/ml) mit 100 mg tPA/ml (Verhältnis CU/μg = 1/4) in aktives Plasmin umgewandelt ist. Die Bildung von Plasmin kann dabei durch entsprechende Erhöhung der Konzentration des Lys-Plasminogens bzw. von tPA beschleunigt werden.

Im Rahmen der vorliegenden Erfindung wird zur Herstellung der erfindungsgemäßen Zusammensetzung oder der erfindungsgemäßen Applikation der Einzelkomponenten zusammen mit einer geeigneten, an den Ort der Thrombose einzuführenden Vorrichtung auch ein Set (Kit) in Form einer Verpackungseinheit bereitgestellt, die insbesondere zur Herstellung des erfindungsgemäßen Präparates kurz vor der systemischen und insbesondere lokalen Applikation zweckmäßig ist. Der Kit besteht mindestens aus 2 Behältern, die jeweils mindestens eine der zur Herstellung des erfindungsgemäßen Präparates oder zur erfindungsgemäßen Applikation erforderlichen Komponente(n) enthalten. Ein Behälter enthält dabei z.B. aktives Plasmin bildende(n) Komponente(n), oder bereits aktives, vorzugsweise stabilisiertes Plasmin, oder das Zymogen des Plasmins, d.h. Plasminogen, vorzugsweise Lys-Plasminogen, und der andere Behälter als zweite Komponente den Plasminogenaktivator, der zumindest teilweise in freier, nicht mit Plasmin komplexierter Form vorliegt.

Der Kit kann aber z.B. auch aus 3 Behältern bestehen, von denen jeder Behälter eine Komponente aus der Gruppe Plasmin, Plasminogenaktivator und Plasminogen(en) enthält.

In einer weiteren Ausführungsform kann der Kit aus 2 Behältern bestehen, von denen einer aktives Plasmin enthält, und der andere die Komponenten Plasminogen(e) und Plasminogenaktivator.

Die Erfindung wird nun durch die nachfolgenden Beispiele näher erläutert, ohne sie darauf zu beschränken.

## Beispiele

### Bestimmung der caseinolytischen Plasminaktivität

Casein wird als 4 %-ige Lösung in einem Phosphatpuffer (67 mM, pH 7,4) bereitgestellt. 2.0 ml Caseinlösung werde mit 1,6 ml des Phosphatpuffers und 0.4 ml der Probe gemischt und 30 Minuten bei 37 °C inkubiert. Danach wird das nicht gespaltene Casein durch Zusatz von 6 ml 15 %-iger Trichloressigsäure ausgefällt. Die Extinktion bei 280 nm wird im Überstand gemessen. Die caseinolytische Aktivität wird nach der folgenden Formel berechnet:

$$E280 \cdot 16{,}3 \cdot \text{Verdünnung der Probe} = CU/ml$$

### Bestimmung der amidolytischen Plasminaktivität

Das chromogene Substrat S 2403 (Firma Chromogenix) wird durch Plasmin amidolytisch gespalten. Dabei wird p-Nitroanilin freigesetzt, welches spektrophotometrisch als Extinktion bei 405 nm gemessen werden kann. Die Meßwerte werden durch Vergleich mit einem Plasminstandard (Plasmin, Fa. Chromogenix) ausgewertet.

### Beispiel 1

Es wird eine erfindungsgemäße Zusammensetzung hergestellt. Gemäß der EP 0353218 hergestelltes Lys-Plasminogen wurde zur Virusinaktivierung gemäß EP 0159311 behandelt, und dann mit tPA (Actilyse, Boehringer Mannheim) in 0.5 mM Tris/NaCl Puffer (pH = 7,4) inkubiert.

In einem Röhrchen wurden jeweils gleiche Mengen von Lösungen der beiden Komponenten in unterschiedlichen Konzentrationen vermischt. Die Konzentration an Lys-Plasminogen in der Inkubationsmischung betrug 1,6, 6,2, 12,5, 25 und 50 CU/ml. Die Menge an tPA wurde so gewählt, daß ein Verhältnis von CU/μg von 1:4 eingestellt wurde. Die Inkubationszeit betrug 2 bzw. 6 min. Danach wurde die Plasminaktivität amidolytisch bestimmt. Die Ergebnisse sind in der nachfolgenden Tabelle eingeführt.

EP 0 674 906 A2

Tabelle

| Herstellung von aktivem Plasmin in Gegenwart von tPA | | | |
|---|---|---|---|
| Lys-Plasminogen (CU/ml) | tPA ($\mu$g/ml) | Plasmin/Lys-Plasminogen (%) nach Inkubationsdauer von | |
| | | 2 min | 6 min |
| 1,6 | 6,4 | 15 | 43 |
| 6,2 | 24,8 | 30 | 69 |
| 12,5 | 50 | 51 | 69 |
| 25 | 100 | 55 | 64 |
| 50 | 200 | 88 | 56 |

**Beispiel 2**

In einem weiteren Versuch wurde das erfindungsgemäße Präparat durch kontinuierliche Inkubation hergestellt. 2500 CU Lys-Plasminogen wurden in 25 ml Ringer-Lösung und 50 mg tPA in 125 ml Ringer-Lösung bereitet. Die Lösungen wurden im gleichen Verhältnis gleichzeitig in einen Katheter eingeleitet, welcher üblicherweise für die medizinische Anwendung von pharmazeutischen Präparaten in Blutgefäßen verwendet wird. Der Katheter war 1,5 m lang und wies ein Volumen von 0.5 ml auf. Die Durchflußrate betrug 30 ml pro Stunde. Nach dem Austritt der Lösung wurde die Plasminaktivität amidolytisch bestimmt.

Die kontinuierliche Herstellung der Präparation wurde über einen Zeitraum von 45 min beobachtet. Es zeigte sich, daß während des gesamten Beobachtungszeitraumes die Umwandlung von Plasminogen in Plasmin in Gegenwart von tPA nahezu vollständig war (98, 6 %).

**Beispiel 3**

Mit einer gemäß Beispiel 1 frisch hergestellten erfindungsgemäßen Zusammensetzung wurde eine Thrombolysetherapie durchgeführt.

In einer Studie wurden 20 Patienten mit Karotis-Embolie durch lokale Verabreichung des erfindungsgemäßen Präparates und im Vergleich dazu mit einem Plasminogenaktivator alleine behandelt. Als erfindungsgemäßes Präparat wurde ein Gemisch aus frisch hergestelltem aktivem Plasmin und tPA (Actilyse, Fa. Boehringer) eingesetzt. Über 2 Infusionspumpen wurden gleichzeitig und parallel jeweils 2500 CU Lys-Plasminogen (IMMUNO AG) und 10 mg tPA pro 25 ml 0,9 %-ige NaCl-Lösung im gleichen Verhältnis einem Mikrokatheter eine Stunde lang zugeführt. Zum Vergleich wurde tPA alleine (20 ml pro 2 Std.) infundiert. Am Ort der Thrombose wurde die Thrombolyse mittels diagnostischer Angiographie in 15 min-Intervallen verfolgt.

Es zeigte sich, daß die erfindungsgemäße Präparation in 8 von 9 Fällen nach durchschnittlich 45 min. eine vollständige Thrombolyse bewirkte. Der Vergleichsversuch mit alleiniger Verwendung von tPA zeigte trotz einer doppelt so langen Behandlungsdauer, daß nur in 8 von 11 Fällen die Lyse vollständig war. Die durchschnittliche Lysezeit betrug 103 min, war also mehr als doppelt so lang.

**Patentansprüche**

1. Pharmazeutische Zusammensetzung mit thrombolytischer Wirkung, enthaltend Plasmin und einen Plasminogenaktivator, der zumindest teilweise in freier Form, also nicht als Komplex mit Plasmin und/oder Plasminogen vorliegt, gegebenenfalls zusammen mit üblichen pharmazeutischen Hilfs- und/oder Trägerstoffen.

2. Zusammensetzung nach Anspruch 1, dadurch gekennzeichnet, daß der Plasminogenaktivator zu mindestens 30 %, vorzugsweise mindestens 50 %, in freier Form, vorliegt.

3. Zusammensetzung nach Anspruch 1 oder 2, dadurch gekennzeichnet, daß das Plasmin ein fibrinolytisch aktives Plasmin ist.

7

4. Zusammensetzung nach einem der Ansprüche 1 bis 3, dadurch gekennzeichnet, daß das Plasmin ein aus einem Plasminogen in vitro erhaltenes Plasmin ist.

5. Zusammensetzung nach einem der Ansprüche 1 bis 4, dadurch gekennzeichnet, daß das Plasmin in stabilisierter Form vorliegt.

6. Zusammensetzung nach einem der Ansprüche 1 bis 5, dadurch gekennzeichnet, daß die Zusammensetzung zusätzlich noch ein oder mehrere Plasminogene enthält.

7. Zusammensetzung nach Anspruch 6, dadurch gekennzeichnet, daß die Zusammensetzung zusätzlich Lys-Plasminogen enthält.

8. Zusammensetzung nach einem der Ansprüche 1 bis 7, dadurch gekennzeichnet, daß der Plasminogenaktivator ein oder mehrere Plasminogenaktivatoren umfaßt, die ausgewählt sind aus der Gruppe bestehend aus tPA, Urokinase, Pro-Urokinase und Streptokinase.

9. Zusammensetzung nach einem der Ansprüche 1 bis 8, dadurch gekennzeichnet, daß das molare Verhältnis Plasmin/Plasminogenaktivator 1/0.01 bis 1/3 beträgt.

10. Zusammensetzung nach einem der Ansprüche 1 bis 9, dadurch gekennzeichnet, daß die Zusammensetzung als Plasminogenaktivator mindestens tPA enthält.

11. Zusammensetzung nach einem der Ansprüche 1 bis 10, dadurch gekennzeichnet, daß die Zusammensetzung Plasmin und tPA im Verhältnis 1:0.1 bis 1:1000, vorzugsweise 1:0.5 bis 1:125 und insbesondere von ca. 1:10 CU/$\mu$g enthält.

12. Zusammensetzung nach einem der Ansprüche 6 bis 11, dadurch gekennzeichnet, daß das molare Verhältnis von Plasmin zu Plasminogenen 1:0.01 bis 1:1 beträgt.

13. Verwendung einer Zusammensetzung nach einem der Ansprüche 1 bis 12 zur systemischen oder lokalen Applikation bei der Behandlung von Thrombose und zur Prophylaxe von Embolien.

14. Verwendung von Plasmin zusammen mit Plasminogenaktivator zur Herstellung eines Arzneimittels zur systemischen oder lokalen Applikation bei der Behandlung von Thrombose und zur Prophylaxe von Embolien.

15. Verwendung einer Zusammensetzung nach einem der Ansprüche 1 bis 12 oder von Plasmin und Plasminogenaktivator zur Herstellung eines Applikationssystems zur lokalen Applikation bei der Behandlung und Prophylaxe von Thrombose und ähnlichen Erscheinungsformen.

16. Verwendung nach einem der Ansprüche 13 bis 15, dadurch gekennzeichnet, daß zusätzlich zum Plasmin und Plasminogenaktivator eine oder mehrere Plasminogene eingesetzt werden.

17. Verwendung nach einem der Ansprüche 13 bis 16, dadurch gekennzeichnet, daß man zur Applikation eine Vorrichtung in Form einer in ein Blutgefäß einführbaren Kanüle oder eines Katheters verwendet.

18. Verfahren zur Herstellung einer Zusammensetzung nach einem der Ansprüche 1 bis 12, dadurch gekennzeichnet, daß man die Komponenten Plasminogen und/oder Plasmin mit einem Plasminogenaktivator vermischt und gegebenenfalls inkubiert, um Plasmin zu bilden.

19. Verfahren nach Anspruch 18, dadurch gekennzeichnet, daß die Vermischung in einem geeigneten Träger, gegebenenfalls zusammen mit anderen üblichen pharmazeutischen Hilfsstoffen erfolgt.

20. Verfahren nach Anspruch 18 oder 19, dadurch gekennzeichnet, daß man die Zusammensetzung nach der Vermischung durch Zugabe eines geeigneten Stabilisators stabilisiert.

21. Verfahren nach einem der Ansprüche 18 bis 20, dadurch gekennzeichnet, daß man die Zusammensetzung nach der Stabilisierung durch an sich bekannte Verfahren in die gewünschte Dareichungsform überführt.

22. Verfahren nach Anspruch 21, dadurch gekennzeichnet, daß man die Zusammensetzung lyophilisiert.

23. Verfahren nach einem der Ansprüche 18 bis 22, dadurch gekennzeichnet, daß die Herstellung der Zusammensetzung erst kurz vor der beabsichtigten Applikation im Rahmen einer Thrombolysetherapie oder während der Thrombolysetherapie erfolgt.

24. Verfahren nach einem der Ansprüche 18 bis 23, dadurch gekennzeichnet, daß man die Vermischung der Komponenten in einer an den Ort der Thrombose einzuführenden Vorrichtung vornimmt.

25. Verfahren nach Anspruch 24, dadurch gekennzeichnet, daß man die Vermischung durch gleichzeitige oder hintereinander erfolgende Hindurchführung der Komponenten, gegebenenfalls in einem geeigneten Verdünnungsmittel, durchführt.

26. Zusammensetzung, Verwendung und/oder Verfahren nach einem der vorhergehenden Ansprüche, dadurch gekennzeichnet, daß man Virus-inaktivierte Plasminogene einsetzt.

27. Verfahren zur Herstellung eines Plasmins für eine Zusammensetzung, Verwendung oder ein Verfahren nach einem der Ansprüche 1 bis 26, dadurch gekennzeichnet, daß man das Plasmin aus Plasminogen und einem Plasminogenaktivator in vitro herstellt.

28. Verfahren nach Anspruch 27, dadurch gekennzeichnet, daß das Plasminogen Lys-Plasminogen ist.

29. Verfahren nach einem der Ansprüche 27 oder 28, dadurch gekennzeichnet, daß man das Plasminogen mit dem Plasminogenaktivator im Kontakt bringt und bis zum Erreichen der gewünschten Plasminaktivität inkubiert.

30. Verfahren nach Anspruch 29, dadurch gekennzeichnet, daß man ein Lys-Plasminogen mit einem Plasminogenaktivator bei einer Temperatur von 15 bis 37 °C mindestens während 30 sec., und vorzugsweise mindestens 1 Minute lang inkubiert.

31. Verfahren zur Behandlung von thrombotischen Erscheinungen bei Säugern, dadurch gekennzeichnet, daß man eine Zusammensetzung nach einem der Ansprüche 1 bis 13 oder die Komponente Plasmin, Plasminogenaktivator und gegebenenfalls Plasminogen(e) den unter thrombotischen Erscheinungen leidenden Säugern systemisch oder an den Ort der Thrombose appliziert.